# EUROPEAN PATENT SPECIFICATION

(11) **EP 2 407 255 B1**
(45) Date of publication and mention of the grant of the patent: **16.01.2013**
(21) Application number: 09764267.2
(22) Date of filing: 23.10.2009
(51) Int. Cl.: B09B 3/00, B03B 9/06, C02F 11/04

(54) **METHOD FOR OPERATING A FACILITY FOR THE BIOMETHANISATION OF SOLID ORGANIC WASTE AND FACILITY FOR SAME**
VERFAHREN ZUM BETRIEB EINER ANLAGE ZUR BIOMETHANISIERUNG VON FESTEN ORGANISCHEN ABFÄLLEN UND ANLAGE DAFÜR
PROCÉDÉ D'EXPLOITATION D'UNE INSTALLATION DE BIOMÉTHANISATION DE RÉSIDUS SOLIDES ORGANIQUE ET INSTALLATION PERMETTANT LA MISE EN OEUVRE DE CE PROCÉDÉ

(30) Priority: 12.03.2009 ES 200900699
(43) Date of publication of application: 18.01.2012
(73) Proprietor: Romero Batallan, Carlos, 28220 Majadahonda (ES)
(72) Inventor: Romero Batallan, Carlos, 28220 Majadahonda (ES)
(74) Representative: Arpe Fernandez, Manuel de
(86) International application number: PCT/ES2009/070455
(87) International publication number: WO 2010/103138

(56) References cited:
- WO-A-98/32341
- ES-A1- 2 261 048
- JP-A- 4 326 994
- US-A- 5 100 066
- US-A1- 2008 020 456
- US-A1- 2008 035 561

## Description

### Scope and prior art

The invention can be applied to the management of urban solid waste (USW), including the production of biogas, such process being commonly known in the art as biomethanization.

Gas is generated during the decomposition of organic substances, and such gas can be used as an alternative source of energy. Such gas, considering its origin, is commonly known as biogas. One of the most important components of biogas is methane, generated from the putrefaction and decomposition, under anaerobic conditions, of organic fractions of animal and vegetable origin.

According to the current state of the art, as reflected for example from document US2008/0020456A1 one of the best known methods is the processing of urban solid waste using anaerobic fermentation bioreactors such fermentation being carried out by means of methanogenic bacteria. After the fermentation, the organic pulp, commonly known as "Digested slurry", is subject to a centrifugation process to obtain a liquid fraction, which is rejected, and a solid fraction, which is deposited on composting beds for its aerobic fermentation and its subsequent transformation into an organic soil conditioner or "compost" which can be used as soil fertilizer and/or in the forestry industry (forest manure). However, this entails a risk of explosion, since this solid fraction obtained at the bioreactor through the centrifuge is still subject to anaerobic fermentation, as a result of the anaerobic bacteria that are still present. However, it undergoes an aerobic condition, when it is conveyed on non-waterproof belts from the bioreactor to the centrifuge, and from this latter one to the composting beds. This way, the solid fraction gradually captures oxygen and might reach a residence condition at the conveyor belt with an oxygen content ranging approximately between 6% and 8%, as well as methane (firedamp). Under certain conditions, this gas mixture might entail a danger of explosion.

Additionally, document ES 2261048 A1 reveals a method and a reactor for the fermentation treatment of lixiviates from landfills and plants for the processing of solid urban waste and the use of the resulting liquid as plant fertilizer. The liquid obtained from the lixiviation of urban solid waste with a predetermined COD (Chemical Oxygen Demand) is subject to fermentation with microorganisms to reduce the COD levels to 50% of the initial one. Alternatively, for the fermentation treatment, methanogenic bacteria are inoculated. These bacteria are selected from a group comprising Methanobacterium, Methanobrevibacter, Metanospirillum and Methanosarcine or a mixture of them, so that it is possible to obtain methane (CH₄) from the fermentation of the above-mentioned lixiviates, as an intermediate product, and it is possible to use the remaining fermented lixiviates as a liquid fertiliser.

Furthermore, the document ES 2117593 A1 describes a method to obtain feeding stuff from the "compost" resulting from the processing of urban solid waste and/or organic waste, as well as the use of the feeding stuff thus obtained. In this case, the resulting "compost" is subject to a quantitative and qualitative bacteriological analysis step in connection with the polluting and/or cumulative elements and/or compounds that are eliminated during a second step, by means of physical and chemical operations, until the pre-established maximum values are obtained; subsequently, the product is subject to a series of processing steps, namely dehydration or drying, milling and/or sifting and freeze-drying or sterilization (5) to obtain a basic feeding stuff that can optionally be enriched and/or used to produce a complete feeding stuff.

### Object of the Invention

On the basis of the state of the art previously described, one of the objects of the invention is to develop a method for the operation, without risk of explosion, of a plant for the management of urban solid waste used both to generate biogas and to obtain a solid fraction that can be used as an organic soil conditioner or as the basis for feeding stuff for animals, and a liquid fraction that can be used to generate biogas through a new anaerobic fermentation, using the final liquid remnants as a liquid fertilizer.

This object is achieved by means of the characteristics of claim 1. Other additional advantages will be appreciated from the characteristics shown in the dependent method claims.

A further object of the invention is a plant to carry out the method according to claims 4 and 5.

According to the invention, such method comprises:
- an anaerobic fermentation step of the organic pulp obtained from urban solid waste, with the help of anaerobic bacteria;
- a separation step to obtain a liquid fraction or lixiviate, plus a solid fraction of the residues, obtained from the digested slurry or fermented organic pulp;
- a feedback step to feedback to the fermentation step part of the liquid fraction as the inoculum of anaerobic bacteria;

Such method being **characterized in that** the following steps have been additionally foreseen:
- a fermentation step of the remaining liquid fraction by means of methanogenic bacteria, to obtain methane;
- a filtering step of the residual liquid of the fermentation step for its subsequent physical and chemical treatment and the consequent use of such liquid as a liquid fertilizer;
- a drying step of the solid fraction obtained after the separation step, in order to neutralize the bacterial flora present at such solid fraction through the application of heat; and
- a milling step of the solid fraction that, when it is conveniently processed, can be used as an organic soil conditioner and/or as a raw material for organic feeding stuff.

According to an additional characteristic of the invention, the drying step is carried out at a temperature of 65 °C or higher.

According to the invention it can be advantageous to carry out an inerting step of the solid fraction with N₂.

Furthermore, the plant according to the invention comprises:
- a bioreactor fed with a load of organic pulp obtained from urban solid waste, to ferment such pulp with the help of anaerobic bacteria;
- a centrifugal separator fed with the digested slurry or organic pulp obtained from the bioreactor, to obtain a liquid fraction or lixiviate and a solid fraction from the remnants;
- a return circuit to feedback part of the liquid fraction to the bioreactor as inoculum of methanogenic bacteria;
- storage tanks for the rest of such liquid fraction;
- fermentation tanks, by means of methanogenic bacteria, to obtain methane from the remaining liquid fraction of the storage tanks;
- a filter for the filtering of the residual liquid of the fermentation tanks and its subsequent physical and chemical treatment, to use such liquid as liquid fertilizer;
- a furnace or drying equipment to dry the solid fraction obtained at the centrifugal separator to neutralize through heating the bacterial flora present at such solid fraction; and
- a mill for the milling of the already dried solid fraction (that, after the adequate processing can be used as an organic soil conditioner and/or as raw material for organic feeding stuff; and
- if appropriate, an inerting device to neutralize the solid fraction by means of N₂; and
- if appropriate, a gas holder for the storage and subsequent use ob the gas obtained through the fermentation of the remaining liquid fraction obtained at the fermentation tanks.

According to the invention, the plant advantageously comprises a power generation device fed with the biogas contained at the gas holder, to supply power for the furnace used during the drying of the solid fraction.

### Brief description of the drawings

Other characteristics and advantages of the invention will become apparent from the following description, used in conjunction with the attached drawings, which refer to an exemplary and non limitative embodiment of the invention, and where:

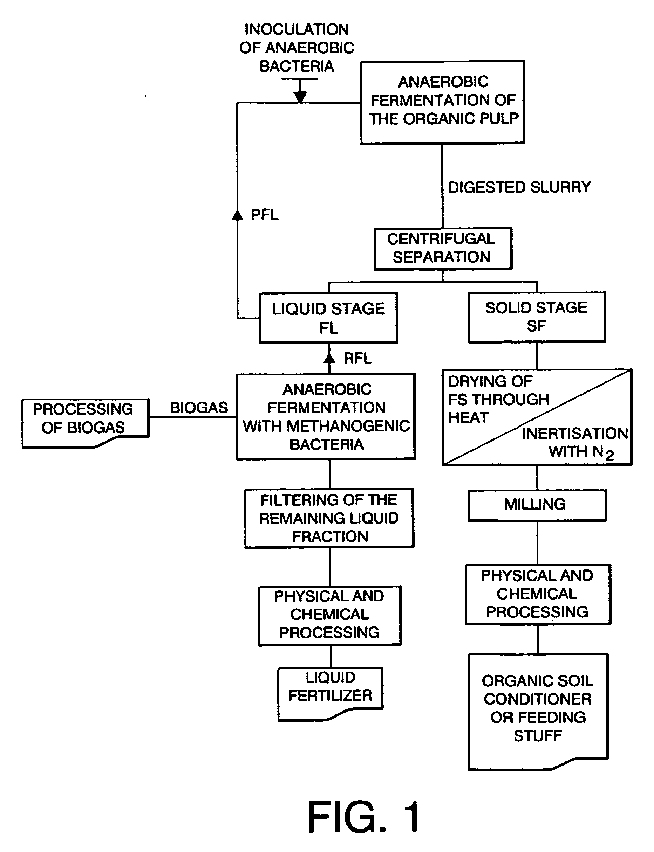

Figure 1 shows a flow diagram of the method according to the invention.

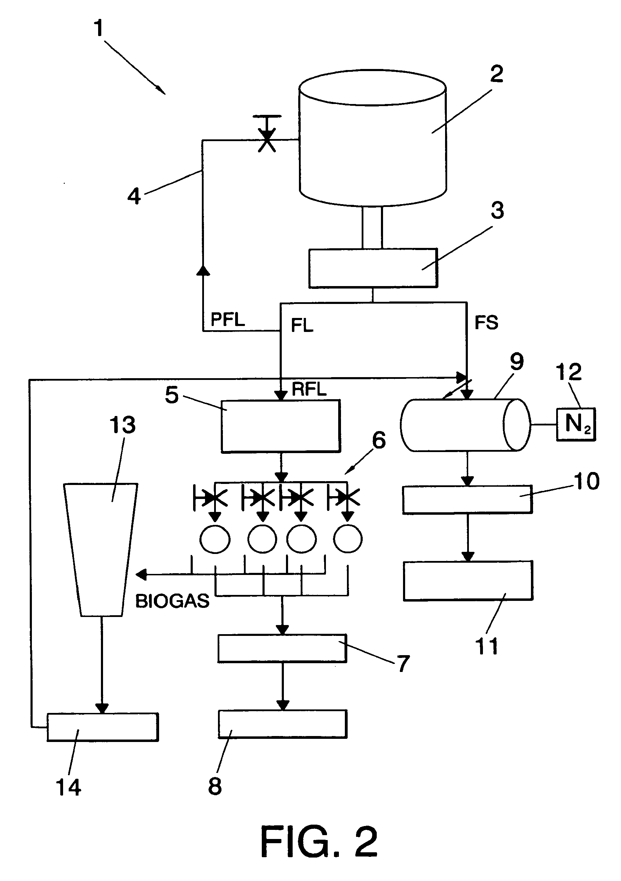

Figure 2 shows a block diagram of the plant to carry out the method according to the invention.

### Detailed description of a preferred embodiment

As it can be seen in the figures, the plant comprises a bioreactor 2 fed with a load of organic pulp obtained from urban solid waste and where an anaerobic fermentation occurs with the help of anaerobic bacteria;

Subsequently, the digested slurry or organic pulp obtained at the bioreactor is fed to a centrifugal separator 3 to obtain a solid fraction or lixiviate LF and a solid fraction SF obtained from the remnants.

A return circuit 4 can be foreseen to feedback part of the liquid fraction PLF to the bioreactor 2 as inoculum of bacteria.

The rest of the liquid fraction LF is kept in storage tanks 5 and is subject to anaerobic fermentation in fermentation tanks 5 by means of methanogenic bacteria, to obtain methane.

The residual liquid obtained from this fermentation is filtered through filters 7 and stored in vessels 8 for its subsequent physical and chemical processing, in order to use such liquid as a liquid fertilizer.

In connection with the processing of the liquid fraction, as it has been previously mentioned, reference is made herein to document ES 2261048 mentioned above, registered in the name of the applicant.

The solid fraction SF obtained at the centrifugal separation device is subject to a drying process at a furnace 9 to neutralize through heating, preferably at a temperature of 65 °C or higher, the anaerobic bacterial flora still present at the solid fraction.

The dried solid fraction, i.e., the fraction without the biota, is fed to a mill 10 and then it can be stored at silos or packaged 10 for its subsequent processing and use as an organic soil conditioner and/or as a raw material for organic feeding stuff.

To make use of such solid fraction, we must mention herein document ES 2117593, also registered in the name of the applicant.

If appropriate, an additional inerting method of such solid fraction can also be implemented by means of N₂, with the help of a device 12.

If appropriate, the biogas generated at the fermentation tanks 6 can be sent to a gas holder 13 for its subsequent use, for instance, to produce power through a power generating device 14, such power being used to heat the furnace 9.

As it will be easily understood by any person skilled in the art, the above is a merely illustrative example of a preferred embodiment of the invention. Therefore, any kind of technical modifications are possible.

Once the object of the invention has been described sufficiently in detail, it must be stated that the embodiments derived from any change in the shape, the size or similar, as well as those derived from any application of what has been previously been disclosed, should also be considered included within the scope of the invention, so that it will only be limited by the scope of the following claims.

## Claims

1. A method to obtain a plant for the conversion of organic solid waste into biomethane, comprising:
- an anaerobic fermentation step of the organic pulp obtained from urban solid waste, with the help of anaerobic bacteria;
- a separation step to obtain a liquid fraction or lixiviate (LF), plus a solid fraction (SF) of the residues, obtained from the digested slurry or fermented organic pulp;
- a feedback step to feedback to the fermentation step part of the liquid fraction (PLF) as the inoculum of anaerobic bacteria;
Such method being **characterized in that** the following steps have been additionally foreseen:
- a storage and fermentation step of the remaining liquid fraction (RLF) by means of methanogenic bacteria, to obtain methane;
- a filtering step of the residual liquid of the fermentation step for its subsequent physical and chemical treatment and the consequent use of such liquid as a liquid fertilizer;
- a milling step of the solid fraction (SF) that, when it is conveniently processed, can be used as an organic soil conditioner and/or as a raw material for organic feeding stuff;
- a drying step of the solid fraction (SF) obtained after the separation step, in order to neutralize the bacterial flora present at such solid fraction through the application of heat.

2. A method according to claim 1, **characterized in that** the drying step is carried out at a temperature of 65 °C or higher.

3. A method according to claim 1, **characterized in that** it is additionally foreseen to carry out an inerting step of the solid fraction (SF) with N₂.

4. Plant to carry out the method according to claims 1 to 3, such plant (1) comprising:
- a bioreactor (2) fed with a load of organic pulp obtained from urban solid waste, to ferment such pulp with the help of methanogenic bacteria;
- a centrifugal separator (3) fed with the digested slurry or organic pulp obtained from the bioreactor, to obtain a liquid fraction or lixiviate (LF) and a solid fraction (SF) from the remnants;
- a return circuit (4) to feedback part of the liquid fraction (PLF) to the bioreactor as inoculum of methanogenic bacteria;
**characterized in that** such plant additionally include:
- storage tanks (5) for the rest of the liquid fraction (RLF);
- fermentation tanks (6), by means of methanogenic bacteria, to obtain methane from the remaining liquid fraction of the storage tanks;
- filters (7) for the filtering of the residual liquid contained at the fermentation tanks and its subsequent physical and chemical treatment, to use such liquid as liquid fertilizer;
- a tank (8) to store the filtered residual liquid;
- a furnace (9) to dry the solid fraction (FS) obtained at the centrifugal separator to neutralize the bacterial flora present at such solid fraction by heating; and
- a mill (10) for the milling of the already dried solid fraction (DSF) that, after the adequate processing can be used as an organic soil conditioner and/or as raw material for organic feeding stuff; and
- if appropriate, an inerting device (12) to neutralize the solid fraction (FS) by means of N₂; and
- if appropriate, a gas holder (13) for the storage and subsequent use ob the gas obtained through the fermentation of the remaining liquid fraction obtained at the fermentation tanks (6).

5. A plant according to claim 4, **characterized in that** additionally, a power generation device (14) has been foreseen, fed with the biogas contained at the gas holder, to supply heating power for the drying furnace (9) used with the solid fraction.

## Patentansprüche

1. Eine Verfahren für die Erstellung einer Anlage für die Umwandlung von organischem, festem Abfall in Biomethan umfassend:
- Eine anaerobe Fermentationsphase der organischen Masse, die mit Hilfe von anaeroben Bakterien aus Hausmüll gewonnen wurde.
- Eine Trennphase zwecks Erhalt der flüssigen Fraktion oder des Auslaugungsproduktes (LF), und einer Feststofffraktion (SF), die aus dem Faulschlamm oder der organischen, fermentierten Masse gewonnen wird.
- Eine Rückführungsphase zur Rückführung der Fermentationsphase der flüssigen Fraktion (PLF) als Impfstoff der anaeroben Bakterien.
Wobei diese Verfahren **dadurch gekennzeichnet ist, dass** folgende zusätzlichen Etappen vorgesehen sind:
- Eine Lagerungs- und Fermentationsphase der restlichen, flüssigen Fraktion (RLF) mittels methanogenen Bakterien zwecks Gewinnung von Methan;
- Eine Filterungsphase des flüssigen Rückstandes der Fermentationsphase zwecks darauffolgender physischer und chemischer Aufbereitung, indem besagte Flüssigkeit als Flüssigdünger verwendet wird.
- Eine Zerkleinerungsphase des Feststofffraktion (SF), die nach einer entsprechenden Aufbereitung als Stabilisierer für organische Böden und/oder als Rohstoff für die Produktion von organischem Futter verwendet werden kann;
- Eine Trocknungsphase der nach der Trennphase erhaltenen festen Fraktion (SF) zwecks Neutralisierung der in dieser festen Fraktion vorhandenen bakteriellen Flora durch Anwendung von Hitze;

2. Eine Verfahren übereinstimmend mit Anspruch eins, **dadurch gekennzeichnet, dass** die Trockungsphase bei einer Temperatur von 65°C oder mehr durchgeführt wird.

3. Eine Verfahren übereinstimmend mit Anspruch 1, **dadurch gekennzeichnet, dass** zusätzlich die Durchführung einer Inertisierungsphase der festen Fraktion (SF) mit N₂ vorgesehen ist.

4. Eine Anlage für die Durchführung der Verfahren übereinstimmend mit den Ansprüchen eins bis drei, wobei diese Anlage (1) folgendes umfasst:
- einen Bioreaktor (2), der mit einer Ladung aus festem Hausmüll gewonnener, organischer Masse beschickt wird, zwecks Fermentation dieser Masse mit Hilfe von methanogenen Bakterien.
- eine Zentrifugtrennungseinrichtung (3), die mit dem Faulschlamm oder der aus dem Bioreaktor erhaltenen organischen Masse beschickt wird, zwecks Gewinn aus dem Müll einer flüssigen Fraktion oder Lixiviat (LF) und einer festen Fraktion (SF).
- einen Rückführkreislauf (4) für die Rückführung eines Teils der flüssigen Franktion (PLF) zum Bioreaktor als Impfstoff der methanogenen Bakterien.
**dadurch gekennzeichnet, dass** diese Anlage ausserdem folgendes umfasst:
- Lagerbehälter (5) für den Rest der flüssigen Fraktion (RLF);
- Fermentationsbecken (6) mittels methanogenen Bakterien zwecks Gewinn von Methan ausgehend von der restlichen flüssigen Fraktion der Lagerbehälter;
- Filter (7) für die Filterung der Restflüssigkeit in den Fermentationsbecken und zur darauffolgenden physischen und chemischen Aufbereitung für den Gebrauch dieser Flüssigkeit als Flüssigdünger.
- einen Behälter (8) für die Lagerung der gefilterten Restflüssigkeit;
- einen Ofen (9) für das Trocknen der festen, in der Zentrifugtrennungseinrichtung erhaltenen Fraktion (FS) zwecks Neutralisierung der bakteriellen Flora in besagter festen Franktion durch Erhitzung und
- eine Mühle (10) für die Zerkleinerung der festen, schon trockenen Fraktion (DSF), die nach der entsprechenden Aufbereitung als Stabilisierer für organische Böden und/oder als Rohstoff für organisches Futter verwendet werden kann, und
- gegebenenfalls eine Inertisierungsvorrichtung (12) zur Neutralisierung der festen Franktion (FS) mit Hilfe von N₂, und
- gegebenenfalls ein Gastank (13) für die Lagerung und spätere Verwendung des Gases, das durch die Fermentation der restlichen in den Fermentationsbecken (6) erhabenen flüssigen Franktion gewonnenen wurde.

5. Eine Anlage übereinstimmend mit Anspruch 4, **dadurch gekennzeichnet, dass** zusätzlich eine Einrichtung für die Erzeugung von Elektrizität (14) vorgesehen ist, die mit dem Biogas aus dem Gastank gespeist wird, um dem für die feste Fraktion verwendeten Trockenofen (9) Wärmeenergie zuzuführen.

## Revendications

1. Une procédé pour se doter d'une unité de production pour convertir des déchets solides organiques en biométhane, qui comprend:
- Une étape de fermentation anaérobie de la pulpe organique obtenue à partir de déchets solides urbains, avec l'aide de bactéries anaérobies.
- Une étape de séparation pour obtenir une fraction liquide ou lixiviat (LF), ajouté à une fraction solide (SF) des déchets, obtenue à partir de la boue digérée ou de la pulpe organique fermentée ;
- Une étape de rétroalimentation pour rétroalimenter une partie de la étape de fermentation de la fraction liquide (PLF) comme inoculation des bactéries anaérobies.
Cette procédé se **caractérise par** les étapes supplémentaires suivantes qui ont été prévues:
- Une étape de stockage et de fermentation de la fraction liquide restante (RLF) au moyen de bactéries méthanogènes, pour obtenir du méthane ;
- Une étape de filtrage du liquide résiduel de la étape de fermentation pour recevoir ensuite un traitement physique et chimique et l'utilisation postérieure de ce liquide comme fertilisant liquide.
- Une étape de trituration de la fraction solide (SF), celle-ci, après un traitement adéquat, peut être utilisée comme conditionneur de sols organiques et/ou comme matière première afin d'obtenir de l'alimentation organique pour animaux ;
- Une étape de séchage de la fraction solide (SF) obtenue après l'étape de séparation, afin de neutraliser la flore bactérienne présente dans cette fraction solide par l'application de chaleur.

2. Une procédé conformément à la revendication 1, qui se **caractérise par** une étape de séchage s'effectuant à une température de 65 °C ou supérieure.

3. Une procédé conformément à la revendication 1, qui se caractérise parce qu'il a été prévu en plus d'effectuer une étape pour rendre inerte la fraction solide (SF) avec du N2.

4. Une unité de production afin d'appliquer la procédé conformément aux revendications 1 à 3, cette unité de production comprenant (1):
- Un bioréacteur (2) alimenté par une charge de pulpe organique obtenue à partir de déchets solides urbains, pour que cette pulpe fermente à l'aide de bactéries méthanogènes;
- un séparateur par centrifugation (3) alimenté par la boue digérée ou la pulpe organique obtenue à partir du bioréacteur, afin d'obtenir une fraction liquide ou lixiviat (LF) et une fraction solide (SF) à partir des déchets ;
- un circuit de retour (4) pour la rétroalimentation d'une partie de la fraction liquide (PLF) au bioréacteur comme inoculation des bactéries méthanogènes ;
- qui se caractérise parce que cette unité de production inclut en plus:
- des réservoirs de stockage (5) pour le reste de la fraction liquide (RL;);
- des réservoirs de fermentation (6), au moyen de bactéries méthanogènes, afin d'obtenir du méthane à partir de la fraction liquide restante des réservoirs de stockage ;
- des filtres (7) pour le filtrage du liquide résiduel contenu dans les réservoirs de fermentation et son traitement physique et chimique postérieur, afin d'utiliser ce liquide comme fertilisant liquide ;
- un réservoir (8) pour le stockage du liquide résiduel filtré;
- un four (9) pour le séchage de la fraction solide (FS) obtenue dans le séparateur centrifuge, afin de neutraliser la flore bactérienne présente dans cette fraction solide par chauffage; et
- un moulin (10) pour triturer la fraction solide déjà sèche (DSF), celle-ci, après un traitement adéquat, peut être utilisée comme conditionneur de sols organiques et/ou comme matière première pour aliments organiques pour animaux ; et
- le cas échéant, un dispositif pour rendre inerte(12) afin de neutraliser la fraction solide (FS) par du N2; et
- le cas échéant, un réservoir de gaz (13) pour le stockage et l'utilisation postérieure du gaz obtenu par la fermentation de la fraction liquide restante issue des réservoirs de fermentation (6).

5. Une unité de production conformément à la revendication 4, qui se caractérise pour avoir prévu en plus un dispositif de production électrique (14), alimenté par du biogaz contenu dans le réservoir de gaz, afin de fournir l'énergie calorifique au four de séchage (9) utilisé avec la fraction solide.
